# EUROPÄISCHE PATENTSCHRIFT

(11) **EP 3 013 265 B1**
(45) Veröffentlichungstag und Bekanntmachung des Hinweises auf die Patenterteilung: **27.09.2017**
(21) Anmeldenummer: 14732240.8
(22) Anmeldetag: 25.06.2014
(51) Int. Cl.: A61B 17/70, A61B 17/86

(54) **UNRUNDE PEDIKELSCHRAUBE**
OUT-OF-ROUND PEDICLE SCREW
VIS PÉDICULAIRE À FAUX-ROND

(30) Priorität: 27.06.2013 DE 102013106758
(43) Veröffentlichungstag der Anmeldung: 04.05.2016
(73) Patentinhaber: Aesculap AG, 78532 Tuttlingen (DE)
(72) Erfinder: LINDNER, Stephan, 78573 Wurmlingen (DE); BOGAJO, Juan-Jose, 78573 Wurmlingen (DE)
(74) Vertreter: Winter, Brandl, Fürniss, Hübner, Röss, Kaiser, Polte - Partnerschaft mbB
(86) Internationale Anmeldenummer: PCT/EP2014/063401
(87) Internationale Veröffentlichungsnummer: WO 2014/207044

(56) Entgegenhaltungen:
- US-A1- 2008 292 429
- US-A1- 2011 152 948
- US-A1- 2013 253 594

## Beschreibung

Die vorliegende Erfindung betrifft eine Pedikelschraube mit im Querschnitt zumindest abschnittsweise unrundem Schraubenkernprofil.

### Hintergrund der Erfindung

Eine Pedikelschraube ist ein chirurgisches Instrument/Implantat zur Stabilisierung der Wirbelsäule. Die Pedikelschraube wird in der Wirbelsäulenchirurgie häufig bei Stabilisierungsoperationen etwa zur Repositionierung eines Gleitwirbels oder zur Versteifung von Wirbeln verwendet. Ihr in der Regel selbstschneidendes Gewinde erleichtert das Eindrehen der Schraube in die Wirbelbogenwurzeln zweier oder mehrerer Wirbel. Der Schraubenkopf bzw. die Tulpe ist entweder beweglich (polyaxial) über ein Kugelgelenk am Schraubenschaft gelagert oder starr (monoaxial) mit dem Gewinde verbunden.

Im Schraubenkopf/Tulpe befindet sich eine axial sich erstreckende, U-förmige Aussparung, die einen Stab aufnehmen kann. Dieser Stab wird mit einer Setzschraube im Schraubenkopf/Tulpe fixiert. Die Pedikelschraube kann somit als Instrument verwendet werden, um beispielsweise einen versetzten Wirbel (Gleitwirbel) zurück in die richtige Position zu bringen. Zur Stabilisierung der Wirbelsäule werden vier oder mehr Pedikelschrauben mit Stäben entlang der Wirbelsäulenachse verbunden. Pedikelschrauben mit dem vorstehend beschriebenen Aufbau sind im Stand der Technik aus vielen Offenbarungen bekannt, auf die bei der nachfolgenden Beschreibung Bezug genommen wird. Eine erneute detaillierte Beschreibung der Pedikelschraube, insbesondere der Tulpe und Traverse kann daher unter Verweis auf diesen allgemein bekannten Stand der Technik entfallen.

### Stand der Technik

Aus dem Stand der Technik sind jedoch mit Blick auf den Schraubenschaft solche Pedikelschraubenformen bekannt, welche in Schaftlängsrichtung unterschiedliche (kontinuierlich oder schrittweise sich aufweitende) Kernquerschnitte aufweisen. Dadurch soll erreicht werden, dass sich die in die Wirbelbogenwurzel eingedrehte Pedikelschraube insbesondere im äußeren/proximalen Kopfbereich im Bohrloch radial verspannt und somit höhere Kräfte in den Wirbel übertragen kann, ohne sich zu lösen. Auch wurde daran gedacht, die Schraubenflanken in Schaftlängsrichtung hin zum Schraubenkopf kontinuierlich oder stufenweise radial zu erweitern, um so zunehmend in den Wirbelkörper einzuschneiden. Dadurch soll einem Ausbrechen der Pedikelschraube entgegengewirkt werden.

Trotz dieser allgemein bekannten Bemühungen der Bereitstellung eines optimalen Schaft-/Schraubenflanken-Designs bleibt das vordringliche Problem des sicheren Halts der Pedikelschraube im Wirbel über einen langen Zeitraum auch weiterhin bestehen. Insbesondere erweisen sich im Stand der Technik folgende Defekte als besonders schwerwiegend:
- Unzureichende Sekundärstabilität gegenüber auf die Pedikelschraube einwirkenden Rotationskräften,
- Ausreißen der Pedikelschraube aus dem Knochen bei Zug- und/oder Scherkräften,
- Schwergängige und langsame Einschraubbarkeit,
- Unkontrollierbarkeit des Einschraubvorgangs.

Zwar existieren auf dem Gebiet der Schraubentechnik generell eine Vielzahl von unterschiedlichen Schraubenformen und Schraubenkonstruktionen, diese sind aber außerhalb der Medizintechnik auf bestimmte Anwendungen beispielsweise für das Zusammenwirken mit Holz, Kunststoff, Gipsplatten und dergleichen Baumaterialien konzipiert. Knochenmaterial unterscheidet sich hiervon deutlich, nicht nur aufgrund seiner Zusammensetzung und Festigkeit sondern auch aufgrund der Tatsache, dass es sich hierbei um ein noch lebendes Material handelt, das seine Struktur fortlaufend verändert und erneuert. Außerdem sind konventionelle Schrauben außerhalb der Medizintechnik anderen Belastungen (zumeist statische Belastungen) ausgesetzt als bei medizinischer Anwendung im Patientenkörper. Sie können daher aus anderen Materialien hergestellt sein, die eine optimale Verbindung zu dem jeweiligen Baumaterial ergeben. Dies ist in der Medizintechnik aus hygienischen Gründen sowie aus Körperverträglichkeitsgründen nicht oder nur bedingt möglich. Daher können technische Lösungen außerhalb der Medizintechnik nicht ohne Weiteres auf Knochschraubenkonstruktionen übertragen werden. US 2008/0292429 A1 und US 2011 /0152948 A1 offenbaren Knochenschrauben mit im Querschnitt abschnittsweise unrundem Schraubenkernprofil.

### Kurzbeschreibung der Erfindung

Ausgehend von dieser Problematik ist es die Aufgabe der vorliegenden Erfindung, eine Knochen- insbesondere Pedikelschraube bereit zu stellen, mittels der höhere Kräfte in einen Patientenknochen sicher und dauerhaft einleitbar sind. Insbesondere soll die Knochen- bzw. Pedikelschraube vorzugsweise dahingehend optimiert sein, dass eine höhere Sekundärstabilität gegenüber Rotationskräften erreicht wird.

Diese Aufgabe sowie weitere Ziele der vorliegenden Erfindung werden durch eine Knochenschraube mit den Merkmalen des Patentanspruchs 1 gelöst. Vorteilhafte Ausgestaltungen der Erfindung sind Gegenstand der Unteransprüche.

Das Grundprinzip der vorliegenden Erfindung beruht auf der Überlegung, den Gewindeschaft einer Knochen- bzw. Pedikelschraube in zumindest zwei Längsabschnitte zu unterteilen, nämlich einen distalen und einen daran sich (vorzugsweise unmittelbar) anschließenden proximalen Schaftabschnitt. Ausschließlich der distale Schaftabschnitt hat hinsichtlich seines Gewindekerns eine in Längsrichtung des distalen Schaftabschnitts sich erstreckende Querschnittsform, die von einer Kreisform abweicht. Durch diese "unrunde" Querschnittsform des Gewindekerns ausschließlich im distalen Schaftabschnitt kann die Knochen-/Pedikelschraube bestimmte Rotationskräfte mehr aufnehmen als im Fall eines durchgehend kreisförmigen Gewindekerns, ohne sich im Knochen zu lösen oder sich weiter hinein zu drehen. Da der proximale Schaftabschnitt jedoch die Kreisform des Gewindekerns beibehält, kann sich die Knochen-/Pedikelschraube auch weiterhin fest im Patientenknochen insbesondere knochenrandseitig verspannen und so Scherkräfte in den Knochen dauerhaft weiterleiten. Dabei sei vorzugsweise darauf hingewiesen, dass der distale Schaftabschnitt eine bestimmte Abschnittslänge (ca. 0.5 * Gesamtschaftlänge) hat mit einem konstanten kleinen Kerndurchmesser. Der proximale Schaftabschnitt hat ebenfalls eine bestimmte Abschnittslänge (ca. 0.5 * Gesamtschaftlänge) mit einem konstanten großen Kerndurchmesser.

Vorzugsweise ist es vorgesehen, dass die unrunde Kernquerschnittsform ein Polygon mit weiter vorzugsweise scharkantigen Ecken darstellt. Dadurch erhöht sich die Fähigkeit zur Aufnahme/Weiterleitung von Rotationskräften in den Knochen. Es kann vorgesehen sein, dass sich die Anzahl der Ecken im Längsverlauf des distalen Schaftabschnitts ändert. So kann beispielsweise zuerst eine Vierkantform im distalen Bereich ausgebildet sein, die dann in einem Mittenbereich des distalen Schaftabschnitts in eine Fünf- oder Sechskantform übergeht. Dadurch wird die Rotationskraftbelastbarkeit weiter gesteigert. Es kann auch vorgesehen sein, dass sich die Ecken zwischen jeweils längsbenachbarten Schraubengängen in der selben Winkelposition längs des distalen Schaftabschnitts hintereinander anordnen. Alternativ hierzu ist es aber auch möglich, dass die Ecken zwischen jeweils längsbenachbarten Schraubengängen winkelversetzt zueinander angeordnet sind. Im letzteren Fall kann es vorgesehen sein, dass sich die Winkelversatzgröße und/oder die Winkelversatzrichtung in Schaftlängsrichtung kontinuierlich und/oder schlagartig ändert. Durch diese Unregelmäßigkeiten sind größere Rotationskräfte in den Knochen einleitbar und es ergibt sich ein sicherer Halt des Knochenschraube im Knochenmaterial. Außerdem kann der Schraubenschaft und vorzugsweise der distale Schaftabschnitt mit dem unrunden Kerndurchmesser durch einen Gewindewirbelvorgang mittels eines Gewindewirbelwerkzeugs hergestellt sein. Durch diese Maßnahme ergeben sich im Wesentlichen scharfkantige Ecken am Kernumfang, wodurch die Fähigkeit, Rotationskräfte in den Knochen einzuleiten, gesteigert wird.

Vorteilhaft kann es sein, wenn die Außenkanten der Schraubenflanken auch im Bereich des distalen Schaftabschnitts kontinuierlich spiralförmig ausgebildet sind. IInsbesondere kann es vorteilhaft sein, wenn die Außenkanten der Schraubenflanken besonders im distalen Schaftabschnitt knick- und kantenfrei ausgeformt sind. Dadurch wird eine sichere Schraubverbindung zum Knochen gewährleitstet und es können hohe Zugkräfte über die Schraubenflanken in den Knochen eingeleitet werden, ohne dass die Schraube ausreißt.

### Figurenbeschreibung

Die Erfindung wird nachstehend anhand bevorzugter Ausführungsbeispiele unter Bezugnahme auf die begleitenden Figuren näher erläutert.
Fig. 1 zeigt die Perspektivenansicht des Gewindekerns einer Knochenschraube, insbesondere Pedikelschraube gemäß einem ersten bevorzugten Ausführungsbeispiel der Erfindung ohne Gewindeflanke und Tulpe,
Fig. 2 zeigt eine Schnittansicht des Gewindekerns im distalen Schaftabschnitt der Knochenschraube gemäß Fig. 1 ohne Gewindeflanke und Tulpe,
Fig. 3 zeigt den Querschnitt des Gewindekerns im distalen Schaftabschnitt der Knochenschraube gemäß Fig. 1,
Fig. 4 zeigt eine Schnittansicht des Gewindekerns im proximalen Schaftabschnitt der Knochenschraube gemäß Fig. 1 ohne Gewindeflanke und Tulpe,
Fig. 5 zeigt die Perspektivenansicht des Gewindekerns einer Knochenschraube, insbesondere Pedikelschraube gemäß einem zweiten bevorzugten Ausführungsbeispiel der Erfindung ohne Gewindeflanke und Tulpe,
Fig. 6 zeigt die Perspektivenansicht des Gewindekerns einer Knochenschraube, insbesondere Pedikelschraube gemäß einem dritten bevorzugten Ausführungsbeispiel der Erfindung ohne Gewindeflanke und Tulpe
Fig. 7 zeigt in Perspektivenansicht den schematischen Längsverlauf der durch die polygone Kern-Querschnittsform erzeugten Längskanten im distalen Schaftabschnitt und
Fig. 8 zeigt den prinzipiellen Verlauf des Gewindekerns einer Knochenschraube gemäß einem bevorzugten Ausführungsbeispiel der Erfindung.

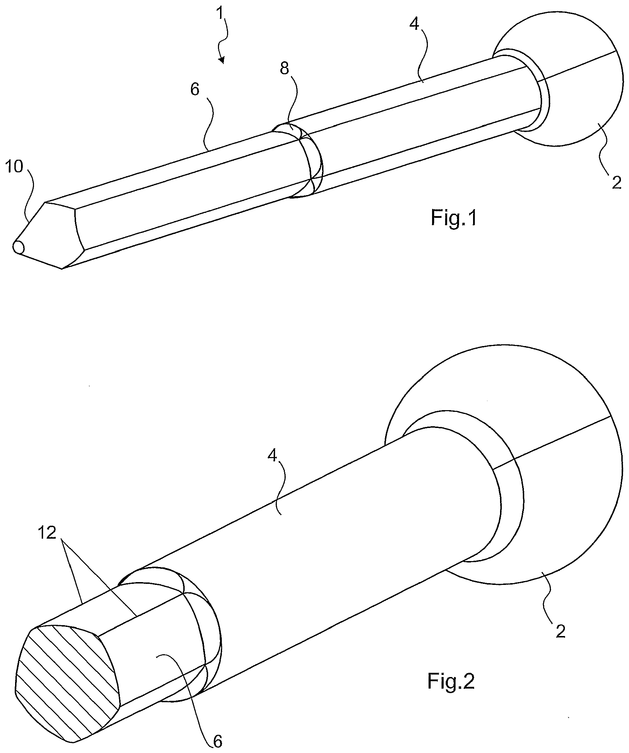

Die in den Fig. 1 bis 3 dargestellte Knochenschraube vorzugsweise in Form einer Pedikelschraube gemäß einem ersten bevorzugten Ausführungsbeispiel der Erfindung hat einen Schraubenschaft 1 und einen kugelkopfartigen Schraubenkopf 2 am proximalen Ende des Schraubenschafts 1. Der Schraubenkopf 2 ist dafür angepasst, mit einer nicht weiter dargestellten zylindrischen Tulpe relativ verschwenkbar gekoppelt zu werden, in der ein axial einseitig offener Längsschlitz ausgehend vom proximalen Tulpenende eingebracht ist, der zum Quereinlegen einer ebenfalls nicht weiter gezeigten Traverse oder Stange vorgesehen ist. Eine solche Tulpenkonstruktion gemäß einer polyaxialen Pedikelschraube ist aus dem Stand der Technik hinlänglich bekannt, sodass an dieser Stelle auf den einschlägigen Stand der Technik verwiesen werden kann. Darüber hinaus sei bereits hier darauf hingewiesen, dass anstelle der gezeigten polyaxialen Pedikelschraube auch eine monoaxiale Pedikelschraube bekannter Ausbildung oder eine einfache Knochenschraube vorgesehen sein kann.

Erfindungsgemäß ist der Schraubenschaft 1 in mehrere, vorzugsweise zwei Längsabschnitte 4, 6 unterteilt. Ein proximaler Längsabschnitt 4 unmittelbar im Längsanschluss an den Schraubenkopf 2 hat vorliegend einen größeren Kerndurchmesser, der zylindrisch ist, d.h. einen in Längsrichtung durchgehend kreisrunden Querschnitt hat. Erfindungsgemäß muss der proximale Längsabschnitt 4 einen über eine bestimmte Schaftlänge konstanten Kerndurchmesser aufweisen.

Die in den Fig. 1 bis 3 nicht gezeigten Schraubenflanken haben in der Regel eine scharfkantige sowie knickfreie Außenkante, wobei die Schraubenflanken-Außendurchmesser dem Kerndurchmesser vorzugsweise folgen. D.h. sofern der Kerndurchmesser variiert, variiert auch der Schraubenflanken-Außendurchmesser dementsprechend. Auf diese Weise ist gewährleistet, dass sich das Schraubengewinde immer ausreichend in den Knochen schneidet und so maximale Zugkräfte in den Knochen einleiten kann.

Der in den Fig. 1 und 2 gezeigte proximale Schaftabschnitt 4 mit zylindrischen Kernquerschnitt geht in einem Mittenbereich 8 des Schraubenschafts 1 in einen distalen Schaftabschnitt 6 über, mit einem Kerndurchmesser, der kleiner ist als der Kerndurchmesser des proximalen Schaftabschnitts 4. Ferner ist der Kerndurchmesser des distalen Schaftabschnitts 6 im vorliegenden Ausführungsbeispiel über die gesamte Abschnittslänge konstant dargestellt.

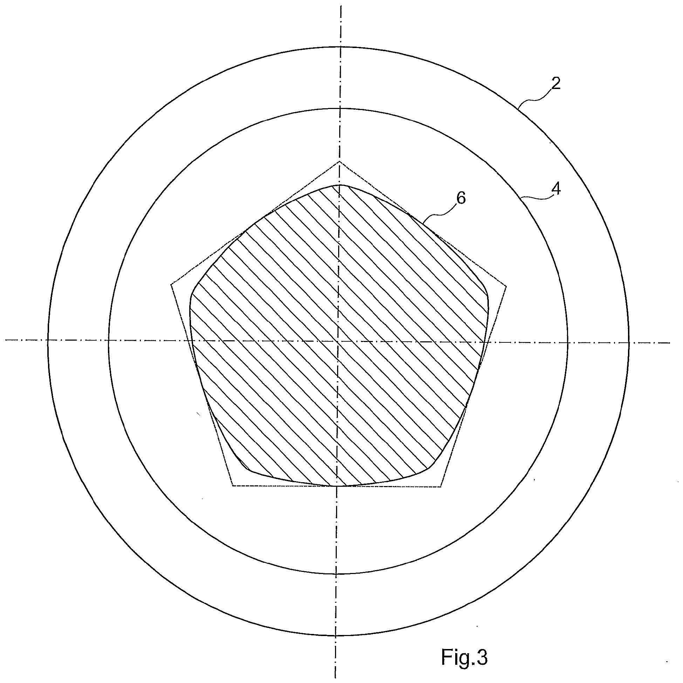

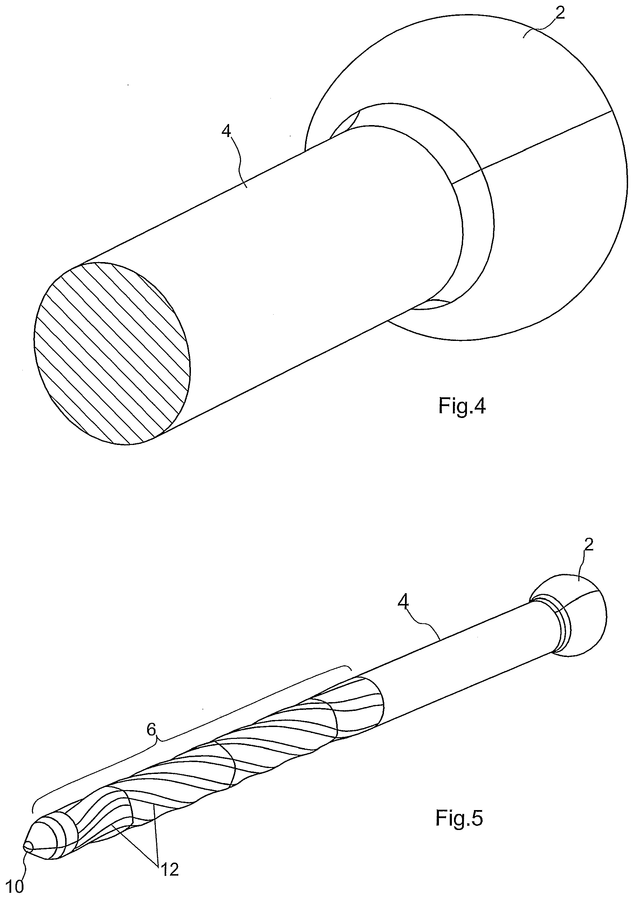

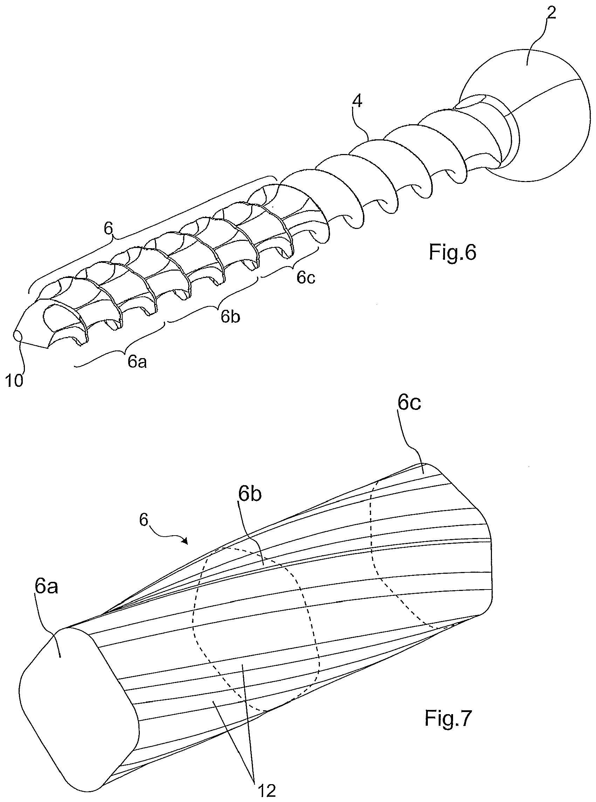

Wichtig bei der vorliegenden Erfindung ist es, dass (ausschließlich) der distale Schaftabschnitt 6 mit einem von der Zylinderform (Kreisform) abweichenden Kernquerschnitt ausgebildet ist. In anderen Worten ausgedrückt, zeigt der kleine Kernquerschnitt des distalen Schaftabschnitts 6 über seine gesamte Länge eine Polygonform vorzugsweise mit gleichen Schenkeln. Insbesondere hat der kleine Kernquerschnitt gemäß der Fig. 3 insgesamt fünf Ecken, wobei diese Polygonform im wesentlichen übergangslos an die Kreisform des proximalen Schaftabschnitts 4 gemäß der Fig. 4 angrenzt. Ferner kann der Außendurchmesser der Schraube insbesondere auch im Bereich des Polygonabschnitts unabhängig von der Polygonform des Schraubenkerns (im Wesentlichen knickfrei) kreisrund sein, wie dies in der Fig. 6 angedeutet ist.

Die fünf Ecken der Polygonform sind gemäß dem ersten bevorzugten Ausführungsbeispiel über die gesamte Länge des distalen Schaftabschnitts immer an der gleichen Winkelposition ausgebildet. Dadurch ergeben sich gemäß der Fig. 2 fünf achsparallele, gerade Außenlinien/Kanten 12. Indessen ist es alternativ gemäß einem zweiten Ausführungsbeispiel der vorliegenden Erfindung auch möglich, die Winkelpositionen der Ecken längs des distalen Schaftabschnitts 6 zu variieren, wie dies insbesondere in der Fig. 5 schematisch dargestellt ist.

Demzufolge sind die Ecken einer polygonen Kernquerschnittsform ausgehend von der Schraubenspitze 10 in Richtung proximal zunächst gegen den Uhrzeigersinn kontinuierlich versetzt um dann in Uhrzeigerrichtung kontinuierlich gegen-versetzt zu werden. Dadurch ergeben sich S- oder Zick-Zack-förmig sich längserstreckende Außenlinien/Kanten 12 am Schraubenkern.

Alternativ oder zusätzlich zu den vorstehend beschriebenen Maßnahmen kann der distale Schaftabschnitt 6 in mehrere Axialbereiche unterteilt sein, in denen die (kleinen, mittleren) Kernquerschnitte jeweils eine Polygonform mit zueinander unterschiedlicher Eckenzahl aufweisen, wie dies in der Fig. 6 dargestellt ist. Demnach kann der distale Schaftabschnitt 6 beispielsweise in drei Axialbereiche 6a, 6b, 6c aufgeteilt sein, wonach die Anzahl der Ecken der jeweiligen Polygonform ausgehend vom der Schraubenspitze 10 von vier auf fünf und dann auf sechs wechselt. Grundsätzlich sind Bereiche mit zwei bis beliebig vielen Ecken denkbar. Dadurch ergibt sich eine Schraubenaußenform des Schraubenkerns, wie diese in der Fig. 7 angedeutet ist.

Um die Polygon-Querschnittsform des Schraubenkerns präzise herstellen zu können, wird erfindungsgemäß ein Gewindewirbelwerkzeug bekannter Konstruktion eingesetzt. Demzufolge wird der "unrunde", vorzugsweise polygone Kernquerschnitt hergestellt, indem das Gewindewirbelwerkzeug innerhalb einer 360° Drehung der Knochenschraube um seine eigene Achse mehrfach in Richtung des Gewindekerns pendelt bzw. verfahren wird. Die dabei erzeugte Pendelbewegung beträgt vorzugsweise zwischen +/- 0.05 mm und +/- 1 mm. Beispielsweise kann das Gewindewirbelwerkzeug (oder alternativ der Gewindekern) in einer bevorzugten Ausführungsform der Erfindung zwischen 3.2mm und 3.7mm Zustellweg pendeln. Dadurch ergeben sich die Ecken zwischen den beiden Zustellwegen, die im Wesentlichen scharfkantig sind, je nach dem, wie schnell die Zustellwege gegenüber der Umdrehungszahl geändert werden.

Im zylindrischen, proximalen Schaftabschnitt 4 wird dann der Gewindekern innerhalb von zwei vollen Umdrehungen der Knochenschraube um seine Achse auf einen Gewindekerndurchmesser beispielsweise von 4mm herausgefahren. Dadurch entsteht der gleichmäßige Übergang 8 vom distalen Schaftabschnitt 6 zum proximalen Schaftabschnitt 4.

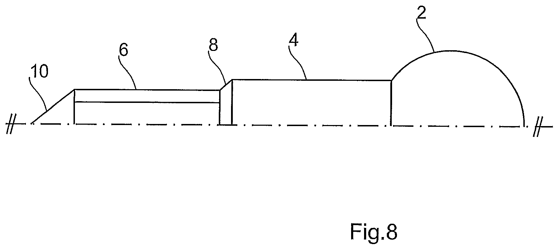

Schließlich ist in der Fig. 8 der Querschnittsverlauf des Schraubenkerns gemäß einem bevorzugten Ausführungsbeispiel der Erfindung prinzipiell dargestellt. Demnach hat die erfindungsgemäße Knochenschraube die Schraubenspitze 10 die kegelförmig in den distalen Schaftabschnitt 6 mit vorliegend konstantem kleinen Kerndurchmesser übergeht. Ausschließlich dieser Abschnitt 6 mit konstant kleinem Kerndurchmesser ist für die Ausbildung der Polygon-Querschnittsform des Schraubenkerns vorgesehen. In einem Mittenbereich des Schraubenschafts bildet sich der Übergangsabschnitt 8 mit kegelförmig sich aufweitendem Kernquerschnitt aus, der in den proximalen Schaftabschnitt 4 mit konstant großem Kerndurchhmesser (Zylinderform) mündet. Dieser proximale Schaftabschnitt 4 zeigt einen Kernquerschnitt mit Kreisform. Offenbart wird zusammenfassend eine medizinische Knochenschraube insbesondere der Pedikelschraubenbauart mit einem Schrauben- und Gewindeschaft 1, der einen distalen Schaftabschnitt 6 mit kleinerem konstantem Kerndurchmesser hat, der in einen proximalen Schaftabschnitt 4 mit größerem konstantem Kerndurchmesser übergeht, an dessen proximalem Ende ein Schraubenkopf 2 ausgebildet ist. Erfindungsgemäß ist die Kernquerschnittsform längs des distalen Schaftabschnitts 6 zumindest teilweise unrund vorzugsweise polygon und die Kernquerschnittsform längs des proximalen Schaftabschnitts 4 kreisrund ausgeformt.

## Patentansprüche

1. Medizinische Knochenschraube insbesondere der Pedikelschraubenbauart mit einem Gewindeschaft (1), der einen distal vordersten Schaftabschnitt (6) mit einem über eine bestimmte Schaftlänge konstanten kleinen Kerndurchmesser hat, und der einen proximal hintersten Schaftabschnitt (4) mit einem über eine bestimmte Schaftlänge konstanten großen Kerndurchmesser hat, an dessen proximalem Ende ein Schraubenkopf (2) angeordnet ist, wobei die Kernquerschnittsform längs des distal vordersten Schaftabschnitts (6) mit über die bestimmte Schaftlänge konstantem kleinen Kerndurchmesser unrund und die Kernquerschnittsform längs des proximal hintersten Schaftabschnitts (4) mit über die bestimmte Schaftlänge konstantem großen Kerndurchmesser kreisrund ausgeformt ist.

2. Medizinische Knochenschraube nach Anspruch 1, **dadurch gekennzeichnet, dass** die unrunde Kernquerschnittsform ein Polygon mit vorzugsweise scharkantigen Ecken ist.

3. Medizinische Knochenschraube nach Anspruch 2, **dadurch gekennzeichnet, dass** sich die Anzahl der Ecken im Längsverlauf des distalen Schaftabschnitts (6) ändert.

4. Medizinische Knochenschraube nach Anspruch 2, **dadurch gekennzeichnet, dass** sich die Ecken zwischen jeweils längsbenachbarten Schraubengängen in der selben Winkelposition längs des distalen Schaftabschnitts (6) hintereinander anordnen.

5. Medizinische Knochenschraube nach Anspruch 2 oder 3, **dadurch gekennzeichnet, dass** die Ecken zwischen jeweils längsbenachbarten Schraubengängen winkelversetzt zueinander angeordnet sind.

6. Medizinische Knochenschraube nach Anspruch 5, **dadurch gekennzeichnet, dass** sich die Winkelversatzgröße und/oder die Winkelversatzrichtung in Schaftlängsrichtung kontinuierlich und/oder schlagartig ändert.

7. Medizinische Knochenschraube vorzugsweise nach einem der vorstehenden Ansprüche, **dadurch gekennzeichnet, dass** der Schraubenschaft (1) zumindest im Bereich eines, einen unrunden Kernquerschnitt aufweisenden distalen Schaftabschnitts (6) durch einen Gewindewirbelvorgang mittels eines Gewindewirbelwerkzeugs hergestellt ist.

8. Medizinische Knochenschraube nach einem der vorstehenden Ansprüche, **dadurch gekennzeichnet, dass** die Außenkanten der Schraubenflanken auch im Bereich des distalen Schaftabschnitts (6) kontinuierlich spiralförmig ausgebildet sind und deren Außenkantendurchmesser dem Kerndurchmesser folgen.

9. Medizinische Knochenschraube nach Anspruch 8, **dadurch gekennzeichnet, dass** die Außenkanten der Schraubenflanken insbesondere im distalen Schaftabschnitt (6) knick- und kantenfrei ausgeformt sind.

10. Medizinische Knochenschraube nach einem der vorstehenden Ansprüche, **dadurch gekennzeichnet, dass** am Schraubenkopf (2) eine hülsenförmige Tulpe zur Aufnahme einer Längsschiene schwenkbar gekoppelt ist.

## Claims

1. A medical bone screw in particular in pedicle screw design, comprising
a threaded shaft (1) including a distal foremost shaft portion (6) having a constant core diameter which is small over a defined shaft length and a proximal rearmost shaft portion (4) having a constant core diameter which is large over a defined shaft length, whose proximal end is provided with a screw head (2), wherein the core cross-sectional shape along the distal foremost shaft portion (6) being formed to be out-of-round along the defined shaft length having the constant, small core diameter and the core cross-sectional shape along the proximal rearmost shaft portion (4) being formed to be circular along the defined shaft length having the constant, large core diameter.

2. The medical bone screw according to claim 1, **characterized in that** the out-of-round core cross-sectional shape is a polygon with preferably sharp-edged corners.

3. The medical bone screw according to claim 2, **characterized in that** the number of the corners changes over the longitudinal extension of the distal shaft portion (6).

4. The medical bone screw according to claim 2, **characterized in that** the corners between respectively longitudinally adjacent screw threads are arranged one behind the other at the same angular position along the distal shaft portion (6).

5. The medical bone screw according to claim 2 or 3, **characterized in that** the corners between respectively longitudinally adjacent screw threads are arranged so as to be angularly staggered with respect to each other.

6. The medical bone screw according to claim 5, **characterized in that** the angular displacement amount and/or the angular displacement direction changes continuously and/or abruptly in the longitudinal direction of the shaft.

7. The medical bone screw preferably according to any one of the preceding claims, **characterized in that** the screw shaft (1) is manufactured by a thread whirling process by means of a thread whirling tool at least in the zone of a distal shaft portion (6) comprising an out-of-round core cross-section.

8. The medical bone screw according to any one of the preceding claims, **characterized in that** the outer edges of the screw flanks, even in the zone of the distal shaft portion (6), are formed like a continuous spiral, their diameters of the outer edges following the core diameter.

9. The medical bone screw according to claim 8, **characterized in that** the outer edges of the screw flanks in particular in the distal shaft portion (6) are shaped so as to have no bends and edges.

10. The medical bone screw according to any of the preceding claims, **characterized in that** a sleeve-shaped tulip for receiving a longitudinal rail is pivotally coupled to the screw head (2).

## Revendications

1. Vis à os médicale, notamment du type vis pédiculaire, comprenant un corps fileté (1), qui possède un tronçon de corps distal avant (6) avec un petit diamètre d'âme constant sur une longueur de corps déterminée, et un tronçon de corps proximal arrière (4) avec un grand diamètre d'âme constant sur une longueur de corps déterminée, à l'extrémité proximale duquel est agencée une tête de vis (2), vis à os médicale
dans laquelle la forme de section transversale de l'âme le long du tronçon de corps distal avant (6) avec un petit diamètre d'âme constant sur la longueur de corps déterminée, est de configuration non circulaire, et la forme de section transversale de l'âme le long du tronçon de corps proximal arrière (4) avec un grand diamètre d'âme constant sur la longueur de corps déterminée, est de configuration ronde circulaire.

2. Vis à os médicale selon la revendication 1, **caractérisée en ce que** la forme de section transversale non circulaire est un polygone de préférence avec des sommets à angle vif.

3. Vis à os médicale selon la revendication 2, **caractérisée en ce que** le nombre des sommets varie le long de l'étendue longitudinale du tronçon de corps distal (6).

4. Vis à os médicale selon la revendication 2, **caractérisée en ce que** les sommets entre des filets de vis respectivement voisins longitudinalement, s'agencent dans la même position angulaire les uns à la suite des autres le long du tronçon de corps distal (6).

5. Vis à os médicale selon la revendication 2 ou la revendication 3, **caractérisée en ce que** les sommets entre des filets de vis respectivement voisins longitudinalement, sont agencés de manière angulairement décalée les uns par rapport aux autres.

6. Vis à os médicale selon la revendication 5, **caractérisée en ce que** la grandeur de décalage angulaire et/ou la direction de décalage angulaire varie de manière continue et/ou de manière brusque dans la direction longitudinale du corps.

7. Vis à os médicale de préférence selon l'une des revendications précédentes, **caractérisée en ce que** le corps de vis (1) est fabriqué, au moins dans la zone d'un tronçon de corps distal (6) présentant une section transversale d'âme non circulaire, par une opération de filetage par tourbillonnage au moyen d'un outil de filetage à tourbillonner.

8. Vis à os médicale selon l'une des revendications précédentes, **caractérisée en ce que** les bords extérieurs des flancs de filet sont d'une configuration en forme d'hélice continue également dans la zone du tronçon de corps distal (6), et leurs diamètres de bord extérieur suivent le diamètre d'âme.

9. Vis à os médicale selon la revendication 8, **caractérisée en ce que** les bords extérieurs des flancs de filet des vis sont, notamment dans le tronçon de corps distal (6), d'une forme exempte de discontinuités et d'arêtes.

10. Vis à os médicale selon l'une des revendications précédentes, **caractérisée en ce qu'**à la tête de vis (2) est couplée de manière pivotante, une tulipe en forme de douille destinée à accueillir une glissière longitudinale.
